# EUROPÄISCHE PATENTANMELDUNG

(11) **EP 0 878 459 A1**
(43) Veröffentlichungstag der Anmeldung: **18.11.1998**
(21) Anmeldenummer: 98107777.9
(22) Anmeldetag: 29.04.1998
(51) Int. Cl.: C07C 61/29, C07C 51/235

(54) **Verfahren zur Herstellung von 9,10-Dihydro-9,10-ethanoanthracen-9-carbonsäuren durch Oxidation der entsprechenden Aldehyde**

(30) Priorität: 12.05.1997 DE 19719843
(71) Anmelder: BAYER AG, 51368 Leverkusen (DE)
(72) Erfinder: Neumann, Karl-Heinz, Dr., 53757 Sankt Augustin (DE); Fiege, Helmut, Dr., 51373 Leverkusen (DE); König, Bernd-Michael, Dr., 51467 Bergisch Gladbach (DE)

(57) **Zusammenfassung**

9,10-Dihydro-9,10-ethanoanthracen-9-carbonsäuren werden durch Oxidation der entsprechenden Aldehyde in besonders vorteilhafter Weise erhalten, wenn man die Oxidation mit Sauerstoff oder einem Sauerstoff enthaltenden Gas und in Gegenwart eines Katalysators und eines organischen Verdünnungsmittels durchführt.

## Beschreibung

9,10-Dihydro-9,10-ethanoanthracen-9-carbonsäuren sind wichtige Zwischenprodukte zur Herstellung von 9,10-Dihydro-9,10-methanoanthracen-9-carbaldehyden, die zu 9-Aminoalkyl-9,10-dihydro-9,10-methanoanthracenen umgesetzt werden können. Die letztgenannten Verbindungen sind als Antidepressiva und Neuroleptika von pharmazeutischer Bedeutung (siehe z.B. DE-A-25 56 143 und Chem. Pharm. Bull. 27 (8), 1806-1812 (1979)).

Es ist bekannt 9,10-Dihydro-9,10-ethanoanthracen-9-carbonsäuren durch Oxidation der entsprechenden 9,10-Dihydro-9,10-ethanoanthracen-9-carbaldehyde mit Jones' Reagenz (CrO₃/H₂SO₄) herzustellen (siehe z.B. Chem. Pharm. Bull. a.a.O.). Der Einsatz von molaren Mengen Chromtrioxid ist jedoch mit Problemen verbunden. Die erforderliche Entsorgung der chromhaltigen Abwässer und deren Aufarbeitung zur Rückgewinnung des Chroms verursachen hohe Kosten. Beim Einsatz größerer Mengen von Chromverbindungen ist auch besonderer Aufwand nötig, um Umweltkontaminationen zuverlässig zu vermeiden. Das gleiche gilt für die allgemein bekannte Oxidation von Aldehyden mit molaren Mengen Manganverbindungen.

Es ist auch zu beachten, daß die Oxidation von Aldehyden, die eine Estergruppe enthalten, insbesondere Acetat oder Propionat, nicht im alkalischen Medium durchgeführt werden kann, da sonst die Gefahr der Esterhydrolyse besteht. Aus diesem Grund sind auch die allgemein bekannten, mit Silber katalysierten Oxidationen hier nicht geeignet.

Eine weitere Gefahr bei der Oxidation von Aldehyden der hier interessierenden Art ist die Decarboxylierung durch Überoxidation, die je nach dem verwendeten Oxidationsmittel in Kombination mit den angewendeten Reaktionsbedingungen zu unerwünschten Nebenprodukten und Ausbeuteminderungen führt.

Es besteht deshalb immer noch das Bedürfnis nach einem Verfahren zur Herstellung von 9,10-Dihydro-9,10-ethanoanthracen-9-carbonsäuren, bei denen keine großen Mengen an Chrom- oder Manganverbindungen eingesetzt werden müssen, nicht zwingend ein alkalisches Medium notwendig ist und trotzdem die gewünschten Carbonsäuren in hohen Ausbeuten und Selektivitäten erhalten werden können.

Es wurde nun ein Verfahren zur Herstellung von 9,10-Dihydro-9,10-ethanoanthracen-9-carbonsäuren der Formel (I) gefunden 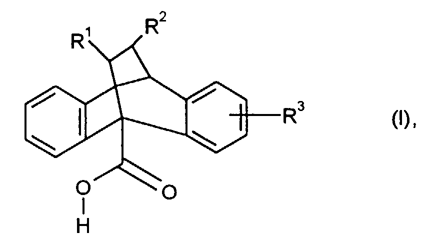 in der
R¹ und R² gleich oder verschieden sein können und jeweils für H, CN, OOCR⁴, COOR⁴ oder (CH₂)ₙR⁵ stehen, wobei
   R⁴ jeweils für C₁-C₁₀-Alkyl, C₃-C₁₀-Cycloalkyl oder C₆-C₁₀-Aryl,
   R⁵ für OH oder CH₂-NH-C₁-C₄-Alkyl und
   n für 1, 2 oder 3 stehen und
R³ für Wasserstoff, Halogen, CN, CHO, COOH, NO₂ oder SO₃H steht,
durch Oxidation von 9,10-Dihydro-9,10-ethanoanthracen-9-carbaldehyden der Formel (II) 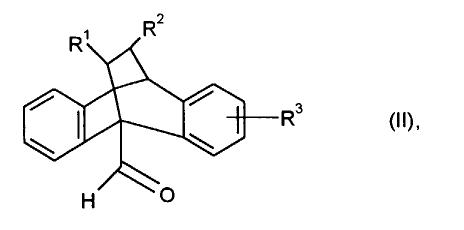 in der die verwendeten Symbole die bei Formel (I) angegebene Bedeutung haben,
das dadurch gekennzeichnet ist, daß man die Oxidation mit Sauerstoff oder einem Sauerstoff enthaltenden Gas und in Gegenwart eines Katalysators und eines organischen Verdünnungsmittels durchführt.

In den Formel (I) und (II) steht vorzugsweise einer der beiden Reste R¹ und R² für H und der andere für CN, OOCR⁴, COOR⁴ oder (CH₂)ₙR⁵.

R³ steht vorzugsweise für Wasserstoff, Fluor, Chlor, Brom oder NO₂.

R⁴ steht vorzugsweise für geradkettiges oder verzweigtes C₁-C₆-Alkyl, C₃-C₇-Cycloalkyl oder Phenyl.

(CH₂)ₙR⁵ steht vorzugsweise für CH₂OH, CH₂CH₂OH oder CH₂-NHCH₃.

Wenn R¹ und/oder R² verschieden ist von H, stehen sie vorzugsweise für OOCR⁴ oder COOR⁴ mit R⁴ = geradkettiges oder verzweigtes C₁-C₄-Alkyl.

Ganz besonders bevorzugt stehen in den Formeln (I) und (II) einer der beiden Reste R¹ und R² für Wasserstoff und der andere für Acetoxy und R³ für Wasserstoff.

Das erfindungsgemäße Verfahren wird mit Sauerstoff oder einem Sauerstoff enthaltenden Gas als Oxidationsmittel durchgeführt. Im einfachsten Fall verwendet man Luft. Man kann auch andere Gemische von Sauerstoff mit Inertgasen verwenden. Solche Gemische enthalten vorzugsweise nicht weniger als 2 Vol.-% Sauerstoff. Bevorzugt sind Sauerstoff und Luft.

Als Katalysatoren können z.B. Salze, Oxide und Komplexverbindungen von Über-gangsmetallen wie Mangan, Kobalt, Eisen, Zirkon, Cer und Chrom in Frage. Mangan, Kobalt und Eisen können dabei z.B. in den Oxidationsstufen +2 und/oder +3, Zirkon in der Oxidationsstufe +4, Cer in den Oxidationsstufen +3 und/oder +4 und Chrom in den Oxidationsstufen +3 und/oder +6 vorliegen. Als Anionen bzw. Komplexliganden können in den Katalysatoren z.B. Acetat, Halogenide, Sulfat, Nitrat oder Acetylacetonat vorhanden sein. Es können auch Wasser enthaltende Katalysatoren eingesetzt werden. Einzelbeispiele für einsetzbare Katalysatoren sind: Mangan(II)-acetat-Hydrat, Mangan(III)-acetat-Hydrat, Eisen(II)-acetat, Kobalt(II)-acetat-Hydrat, Kobalt(III)-acetat-Hydrat, Cer(III)-acetat-Hydrat, Zirkon(IV)-acetat, Chrom(III)-sulfat und die Acetylacetonate dieser Metalle. Besonders bevorzugte Katalysatoren sind Co(CH₃COO)₂ x 4H₂O, Mn(CH₃COO)₂ x 4H₂O, Fe(CH₃COO)₂ und Cr₂(SO₄)₃.

Man kann auch Gemische mehrerer Katalysatoren einsetzen.

Bezogen auf den eingesetzten Aldehyd der Formel (II) kann man beispielsweise 0,005 bis 10 Mol-% Katalysator (berechnet auf Metall) einsetzen. Vorzugsweise beträgt diese Menge 0,07 bis 5 Mol-%, besonders bevorzugt 0,01 bis 3 Mol-%.

Gegebenenfalls können Bromide als Cokatalysatoren eingesetzt werden. Da diese jedoch Korrosionen verursachen können, arbeitet man vorzugsweise ohne derartige Cokatalysatoren.

Als organische Verdünnungsmittel kommen insbesondere Carbonsäuren mit z.B. 1 bis 6 C-Atomen in Frage. Bevorzugt sind Essigsäure und Propionsäure. Man kann beispielsweise, bezogen auf die Summe Verdünnungsmittel + eingesetzter Aldehyd der Formel (II) 1 bis 35 Gew.-% Aldehyd einsetzen. Vorzugsweise beträgt diese Menge 2 bis 20 Gew.-%, insbesondere 3 bis 15 Gew.-%. Es ist nicht erforderlich, daß sich der eingesetzte Aldehyd der Formel (II) vollständig im Verdünnungsmittel löst. Der eingesetzte Aldehyd der Formel (II) kann auch in suspendierter Form vorliegen.

Das erfindungsgemäße Verfahren kann beispielsweise bei Temperaturen im Bereich 15 bis 180°C durchgeführt werden. Bevorzugt sind 20 bis 120°C, insbesondere 30 bis 100°C. Die jeweils optimale Temperatur hängt beispielsweise vom eingesetzten Katalysator ab und kann gegebenenfalls durch einfache Vorversuche leicht ermittelt werden.

Es ist möglich, daß die Oxidation nicht sofort mit dem Einleiten von Sauerstoff oder Sauerstoff enthaltendem Gas beginnt. Es können u.U. Startzeiten von bis zu beispielsweise 3 Stunden auftreten. Gegebenenfalls kann in solchen Fällen eine Temperaturerhöhung vorteilhaft sein. Die Startzeit kann gegebenenfalls auch durch Zusatz einer leicht oxidierbaren Substanz verkurzt werden. Bevorzugt wird für diesen Zweck Acetaldehyd oder Propionaldehyd eingesetzt.

Im Prinzip kann man das erfindungsgemäße Verfahren bei beliebigem Druck durchführen, beispielsweise bei Drücken im Bereich 0,5 bis 20 bar. Bevorzugt arbeitet man bei Normaldruck. Wenn man mit einem relativ niedrig siedenden Verdünnungsmittel bei einer relativ hohen Temperatur arbeiten will, kann die Anwendung von Druck erforderlich sein.

Man kann das erfindungsgemaße Verfahren z.B. so durchführen, daß man den Aldehyd der Formel (II) und das Verdünnungsmittel vorlegt, dann den Katalysator zufügt, die Reaktionstemperatur einstellt und schließlich Sauerstoff oder ein Sauerstoff enthaltendes Gas in das Reaktionsgefäß einleitet.

Den Verlauf der Oxidation kann man z.B. durch Kontrolle des Sauerstoffgehaltes im Abgas bestimmen. Die Reaktion springt an, wenn der Sauerstoffgehalt im Abgas absinkt, und sie läuft so lange, bis der Sauerstoffgehalt wieder den ursprünglichen Wert angenommen hat.

Nach Beendigung der Reaktion kann man gegebenenfalls noch einige Zeit nachrühren.

Die hergestellte 9,10-Dihydro-9,10-ethanoanthracen-9-carbonsäure der Formel (I) kann man aus dem nach der Reaktion vorliegenden Gemisch beispielsweise gewinnen, indem man es auf Raumtemperatur bringt, durch Abziehen von Verdünnungsmittel einengt, mit Wasser versetzt und dann den vorliegenden Niederschlag abfiltriert, wäscht und trocknet.

Mit dem erfindungsgemäßen Verfahren gelingt die Herstellung von 9,10-Dihydro-9,10-ethanoanthracen-9-carbonsäuren der Formel (I) in vergleichbar guter Ausbeute und Selektivität wie bei der Arbeitsweise mit Jones' Reagenz als Oxidationsmittel, jedoch ohne die Nachteile, die bei jener Arbeitsweise in Kauf genommen werden müssen. Auch andere zu erwartende Nachteile treten beim erfindungsgemäßen Verfahren überraschenderweise nicht auf.

### Beispiele

### Beispiel 1

In einem 250 ml-Reaktionsgefäß mit eingeformten Wellenbrechern, Begasungsrührer mit Magnetantrieb, Innentemperaturmessung, Rückflußkühler und Gaszuleitung über die Flüssigkeitsoberfläche wurden 8,8 g 12-Acetoxy-9,10-dihydro-9,10-ethanoanthracen-9-carbaldehyd in 200 ml Essigsäure bei Raumtemperatur suspendiert. Dann wurden 5 mg Kobalt(II)-acetat-Tetrahydrat zugeführt und die Lösung auf 35°C erwärmt. Unter gutem Rühren wurde bei dieser Temperatur 22 l/h Luft in das Reaktionsgefaß eingeleitet. Das Abgas wurde durch den Rückflußkühler und eine Trockenpackung einem O₂-Meßgerät zugeführt. Die Reaktion sprang nach 35 Minuten an. Der O₂-Gehalt des Abgases nahm während der Oxidation bis auf 12,2 Vol.-% ab. Nach etwa 1 Stunde stieg der O₂-Gehalt des Abgases wieder auf 21 Vol.-% an. Es wurde noch 30 Minuten nachgerührt und dann auf Raumtemperatur abgekühlt. Zur Isolierung der 12-Acetoxy-9,10-dihydro-9,10-ethanoanthracen-9-carbonsäure wurde die produkthaltige Essigsäuremischung unter vermindertem Druck auf 50 ml eingeengt und mit 200 ml Wasser versetzt. Das kristalline Produkt wurde abgesaugt, mit 50 ml Wasser gewaschen und an der Ölpumpe getrocknet. Man erhielt 8,5 g 12-Acetoxy-9,10-dihydro-9,10-ethanoanthracen-9-carbonsäure mit einem Gehalt von 93,6 Gew.-% (GC-100 % Methode, syl.). Das Edukt hatte sich vollständig umgesetzt.

### Beispiel 2

In der in Beispiel 1 beschriebenen Apparatur wurden 8,8 g 12-Acetoxy-9,10-dihydro-9,10-ethanoanthracen-9-carbaldehyd und 100 mg Eisen(II)-acetat in 100 ml Essigsäure suspendiert, das Gemisch auf 70°C erwärmt und unter gutem Rühren 14 l/h Luft in den Reaktor eingeleitet. Der O₂-Gehalt des Abgases sank bis auf 8,4 Vol.-% ab. Nach etwa 1,5 h war die O₂-Aufnahme beendet. Es wurde noch 30 Minuten nachgerührt und dann auf Raumtemperatur abgekühlt. Die Reaktionsmischung wurde unter vermindertem Druck auf 50 ml eingeengt, mit 200 ml Wasser versetzt und das kristalline Produkt abgesaugt. Die Kristalle wurden mit 50 ml Wasser gewaschen und an der Ölpumpe getrocknet. Man erhielt 8,5 g 12-Acetoxy-9,10-dihydro-9,10-ethanoanthracen-9-carbonsäure mit einem Gehalt von 87,6 Gew.-% (GC-100 % Methode, syl.). Es verblieb 0,7 Gew.-% Edukt in der Säure.

### Beispiel 3

In der in Beispiel 1 beschriebenen Apparatur wurden 8,8 g 12-Acetoxy-9,10-dihydro-9,10-ethanoanthracen-9-carbaldehyd und 50 mg Mangan(II)-acetat-Tetrahydrat in 100 ml Essigsäure suspendiert. Die Reaktionsmischung wurde auf 45°C erwärmt und unter gutem Rühren 17 l/h Luft in den Reaktor eingeleitet. Der O₂-Gehalt des Abgases sank bis auf 13,0 Vol.-% ab. Nach 45 Minuten war die O₂-Aufnahme beendet. Es wurde noch 30 Minuten nachgerührt und dann auf Raumtemperatur abgekühlt. Die Reaktionsmischung wurde mit 200 ml Wasser versetzt und das kristalline Produkt abgesaugt. Die Kristalle wurden mit 50 ml Wasser gewaschen und an der Ölpumpe getrocknet. Man erhielt 7,7 g 12-Acetoxy-9,10-dihydro-9,10-ethanoanthracen-9-carbonsäure mit einem Gehalt von 89,6 Gew.-% (GC-100 % Methode, syl.). Es verblieb 7,7 Gew.-% Edukt in der Säure.

### Beispiel 4

In der in Beispiel 1 beschriebenen Apparatur wurde die Gasableitung und O₂-Messung entfernt. In der Apparatur wurden 8,8 g 12-Acetoxy-9,10-dihydro-9,10-ethanoanthracen-9-carbaldehyd und 100 mg Kobalt(II)-acetat-Tetrahydrat in 100 ml Essigsäure suspendiert. Die Reaktionsmischung wurde auf 42,5°C erwärmt und unter gutem Rühren 350 ml Sauerstoff in den Reaktor eingeleitet. Nach 10 Minuten war die O₂-Aufnahme beendet. Um die Temperatur zu halten, mußte gut gekühlt werden. Man rührte noch 30 Minuten nach und ließ dann auf Raumtemperatur abkühlen. Die Reaktionsmischung wurde mit 200 ml Wasser versetzt und das kristalline Produkt abgesaugt. Die Kristalle wurden mit 50 ml Wasser gewaschen und an der Ölpumpe getrocknet. Man erhielt 7,5 g 12-Acetoxy-9,10-dihydro-9,10-ethanoanthracen-9-carbonsäure mit einem Gehalt von 91,2 Gew.-% (GC-100 % Methode, syl.). Das Edukt hatte sich vollständig umgesetzt.

### Beispiel 5

In der in Beispiel 1 beschriebenen Apparatur wurden 8,8 g 12-Acetoxy-9,10-dihydro-9,10-ethanoanthracen-9-carbaldehyd und 100 mg Chrom(III)-sulfat in 100 ml Essigsäure suspendiert. Die Reaktionsmischung wurde auf 97°C erwärmt und unter gutem Rühren wurden 20 l/h Luft in den Reaktor eingeleitet. Der O₂-Gehalt des Abgases sank bis auf 13,2 Vol.-% ab. Nach 2,7 Stunden war die O₂-Aufnahme beendet. Man rührte noch 30 Minuten nach und ließ dann auf Raumtemperatur abkühlen. Die Reaktionsmischung wurde unter vermindertem Druck auf 50 ml eingeengt, mit 200 ml Wasser versetrt und das kristalline Produkt abgesaugt. Die Kristalle wurden mit 50 ml Wasser gewaschen und an der Ölpumpe getrocknet. Man erhielt 7,4 g 12-Acetoxy-9,10-dihydro-9,10-ethanoanthracen-9-carbonsäure mit einem Gehalt von 87,7 Gew.-% (GC-100% Methode, syl.). Es verblieben 5,8 Gew.-% Edukt in der Säure.

## Patentansprüche

1. Verfahren zur Herstellung von 9,10-Dihydro-9,10-ethanoanthracen-9-carbonsäuren der Formel (I) 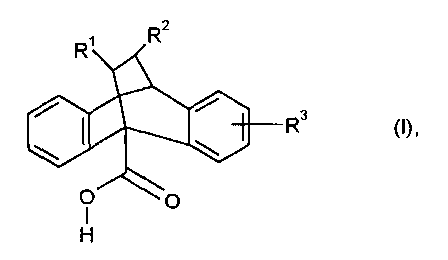 in der
R¹ und R² gleich oder verschieden sein können und jeweils für H, CN, OOCR⁴, COOR⁴ oder (CH₂)ₙR⁵ stehen, wobei
R⁴ jeweils für C₁-C₁₀-Alkyl, C₃-C₁₀-Cycloalkyl oder C₆-C₁₀-Aryl,
R⁵ für OH oder CH₂-NH-C₁-C₄-Alkyl und
n für 1, 2 oder 3 stehen und
R³ für Wasserstoff, Halogen, CN, CHO, COOH, NO₂ oder SO₃H steht,
durch Oxidation von 9,10-Dihydro-9,10-ethanoanthracen-9-carbaldehyden der Formel (II) 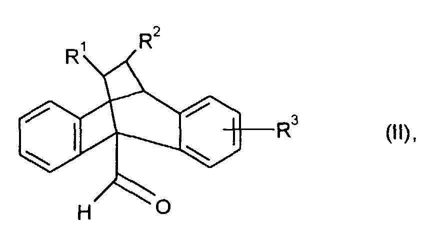 in der die verwendeten Symbole die bei Formel (I) angegebene Bedeutung haben,
dadurch gekennzeichnet, daß man die Oxidation mit Sauerstoff oder einem Sauerstoff enthaltenden Gas und in Gegenwart eines Katalysators und eines organischen Verdünnungsmittels durchführt.

2. Verfahren nach Anspruch 1, dadurch gekennzeichnet, daß in den Formel (I) und (II) einer der beiden Reste R¹ und R² für H und der andere für CN, OOCR⁴, COOR⁴ oder (CH₂)ₙR⁵ stehen.

3. Verfahren nach Ansprüchen 1 und 2, dadurch gekennzeichnet, daß R³ für Wasserstoff Fluor, Chlor, Brom oder NO₂, R⁴ für geradkettiges oder verzweigtes C₁-C₆-Alkyl, C₃-C₇-Cycloalkyl oder Phenyl und (CH₂)ₙR⁵ für CH₂OH, CH₂CH₂OH oder CH₂NHCH₃ stehen.

4. Verfahren nach Ansprüchen 1 bis 3, dadurch gekennzeichnet, daß als Katalysatoren Salze, Oxide und/oder Komplexverbindungen von Übergangsmetallen eingesetzt werden.

5. Verfahren nach Ansprüchen 1 bis 4, dadurch gekennzeichnet, daß bezogen auf den eingesetzten Aldehyd der Formel (II) 0,005 bis 10 Mol-% Katalysator (berechnet auf Metall) eingesetzt wird.

6. Verfahren nach Ansprüchen 1 bis 5, dadurch gekennzeichnet, daß als organische Verdünnungsmittel Carbonsäuren mit 1 bis 6 C-Atomen eingesetzt werden.

7. Verfahren nach Ansprüchen 1 bis 6, dadurch gekennzeichnet, daß bezogen auf die Summe Verdünnungsmittel + eingesetzter Aldehyd der Formel (II) 1 bis 35 Gew.-% Aldehyd eingesetzt wird.

8. Verfahren nach Ansprüchen 1 bis 7, dadurch gekennzeichnet, daß es bei Temperaturen im Bereich 15 bis 180°C durchgeführt wird.

9. Verfahren nach Ansprüchen 1 bis 8, dadurch gekennzeichnet, daß man es durchführt, indem man den Aldehyd der Formel (II) und das Verdünnungsmittel vorlegt, dann den Katalysator zufügt, die Reaktionstemperatur einstellt und dann Sauerstoff oder ein Sauerstoff enthaltendes Gas in das Reaktionsgefäß einleitet.

10. Verfahren nach Ansprüchen 1 bis 9, dadurch gekennzeichnet, daß man die hergestellte Carbonsäure der Formel (I) aus dem nach der Reaktion vorliegender Gemisch isoliert, indem man es auf Raumtemperatur bringt, durch Abzieher von Verdünnungsmittel einengt, mit Wasser versetzt und den dann vorliegenden Niederschlag abfiltriert, wäscht und trocknet.
